# EUROPÄISCHE PATENTSCHRIFT

(11) **EP 3 530 188 B1**
(45) Veröffentlichungstag und Bekanntmachung des Hinweises auf die Patenterteilung: **09.09.2020**
(21) Anmeldenummer: 18178537.9
(22) Anmeldetag: 19.06.2018
(51) Int. Cl.: A61B 6/03, A61B 6/00, H01R 39/00

(54) **COMPUTERTOMOGRAPHIEGERÄT UND VERFAHREN ZUM ERMITTELN EINES BETRIEBSZUSTANDS VON SCHLEIFKONTAKTEN IN EINEM COMPUTERTOMOGRAPHIEGERÄT**
COMPUTER TOMOGRAPHY UNIT AND METHOD FOR DETERMINING AN OPERATING STATE OF GRINDING CONTACTS IN A COMPUTER TOMOGRAPHY UNIT
APPAREIL DE TOMODENSITOMÉTRIE ET PROCÉDÉ DE DÉTERMINATION D'UN ÉTAT DE FONCTIONNEMENT DES CONTACTS GLISSANTS DANS UN APPAREIL DE TOMODENSITOMÉTRIE

(43) Veröffentlichungstag der Anmeldung: 28.08.2019
(73) Patentinhaber: Siemens Healthcare GmbH, 91052 Erlangen (DE)
(72) Erfinder: Gebert, Bernhard, 91083 Hagenau (DE); Krauss, Markus, 91344 Waischenfeld (DE)

(56) Entgegenhaltungen:
- CN-A- 104 545 979
- DE-A1- 10 120 088
- DE-A1-102006 011 968
- JP-A- H07 265 291
- US-A- 5 402 461

## Beschreibung

Die Erfindung betrifft ein Computertomographiegerät und ein Verfahren zum Ermitteln eines Betriebszustands von Schleifkontakten an einem Schleifring für eine Drehübertragung in einem Computertomographiegerät.

Für die Drehübertragung in Computertomographiegeräten werden üblicherweise Schleifringe mit einem oder mehreren Schleifkontakten verwendet. Die Schleifkontakte können beispielsweise in Form von Kohlen und/oder Bürsten realisiert sein. Verschleiß, Verschmutzung und/oder besondere Belastung können einen fehlerhaften Schleifkontakt verursachen. Ein fehlerhafter Schleifkontakt kann einen Lichtbogen verursachen, der den Schleifring beschädigen und insbesondere zu einem Abbruch einer Untersuchung mittels des Computertomographiegeräts führen kann. Da sich die Schleifkontakte mit zunehmender Betriebsdauer abnutzen, müssen sie regelmäßig ausgewechselt werden. Üblicherweise erfolgt die Vermeidung von Beschädigungen der Schleifbahn, die durch Lichtbögen verursacht werden, indem die Schleifkontakte sehr frühzeitig ausgetauscht werden. Das führt zu unnötig hohen Wartungskosten, da dabei auch Schleifkontakte ausgetauscht werden, deren Abnutzung sich noch in einem tolerierbaren Rahmen befindet.

US 5 402 461 A offenbart ein Röntgen-Computertomographiegerät, umfassend einen Drehrahmenabschnitt, ein Röntgenstrahlerzeugungssystem, das an dem Drehrahmenabschnitt angebracht ist, und eine elektrische Energie-übertragungseinrichtung, die aus einem Schleifring und einer Bürste besteht.

DE 101 20 088 A1 offenbart ein Verfahren zum Erfassen von Durchgangsunterbrechungen in einer Schaltung, wobei ein Signal durch eine mit einem leitenden Band eines Schleifrings in Kontakt stehende Bürste übertragen wird, wobei sich die Bürste und der Schleifring relativ zueinander bewegen.

JP H07 265291 A offenbart ein Röntgen-CT-Gerät, aufweisend einen Schleifring, Bürsten aus elektrischem Widerstandsmaterial und Widerstandsmessmittel, wobei ein Bürstenverbrauchsgrad basierend auf einem gemessenen Widerstandswert erfasst werden kann.

CN 104 545 979 A offenbart eine elektrische Bürstendetektionsvorrichtung, wobei ein Drucksensor zu einem elektrischen Bürstenteil hinzugefügt ist, um Druckinformationen zwischen der elektrischen Bürste und einem Gleitring zu sammeln.

DE 10 2006 011 968 A1 offenbart eine Röntgenvorrichtung mit einer Röntgenquelle, mit einem Hochspannungserzeuger und mit einem Inverter zur Erzeugung einer Eingangswechselspannung für den Hochspannungserzeuger, wobei zwischen dem Inverter und dem Hochspannungserzeuger ein Resonanznetzwerk gebildet ist.

Die Erfindung hat die Aufgabe, eine verbesserte Ermittlung eines Betriebszustands von Schleifkontakten an einem Schleifring für eine Drehübertragung in einem Computertomographiegerät zu ermöglichen.

Jeder Gegenstand eines unabhängigen Anspruchs löst diese Aufgabe. In den abhängigen Ansprüchen sind weitere vorteilhafte Aspekte der Erfindung berücksichtigt.

Die Erfindung betrifft ein Computertomographiegerät, aufweisend
- einen Schleifring für eine Drehübertragung, insbesondere für eine Drehübertragung von elektrischer Energie zur Versorgung einer Röntgenquelle,
- eine Zuleitung, wobei der Schleifring relativ zu der Zuleitung drehbar angeordnet ist,
- zwei Schleifkontaktelemente, welche Schleifkontakte zwischen einer Schleifbahn des Schleifrings und der Zuleitung bilden und welche derart zueinander parallel geschaltet sind, dass die zwei Schleifkontaktelemente mittels zumindest eines Abschnitts der Schleifbahn und mittels zumindest eines Abschnitts der Zuleitung miteinander elektrisch leitend verbunden sind,
- eine Messvorrichtung, welche zum Erfassen eines Messwerts, welcher von der resultierenden Induktivität zwischen den zwei Schleifkontaktelementen abhängt, ausgebildet ist,
- eine Ermittlungseinheit, welche zum Ermitteln eines Betriebszustands der Schleifkontakte basierend auf dem Messwert ausgebildet ist.

Dadurch, dass mehrere Schleifkontaktelemente vorgesehen sind, die zueinander parallel geschaltet sind, führt eine Unterbrechung eines der Schleifkontakte nicht ohne weiteres zu einem Spannungsanstieg am Schleifkontaktelement. Daher ist eine Überwachung des Betriebszustands von Schleifkontakten basierend auf einer Messung des Spannungsabfalls über den Schleifkontaktelementen nicht ohne weiteres möglich.

Die Zuleitung kann insbesondere von einem Wechselrichter und/oder von einem Gleichstromzwischenkreis mit elektrischer Energie, beispielsweise in Form zumindest eines hochfrequenten Pulsstroms, gespeist werden. Daher ist eine Überwachung des Betriebszustands von Schleifkontakten, basierend auf einer Messung der Stromverteilung über den Schleifkontaktelementen nicht ohne weiteres möglich. Bei einem Motor oder Generator, in dem weitestgehend sinusförmiger Strom oder Gleichstrom fließt, kann hingegen die Überwachung des Betriebszustands von Schleifkontakten basierend auf einer Messung der Stromverteilung über den Schleifkontaktelementen erfolgen. Insbesondere kann damit der Widerstand eines Schleifkontakts ermittelt werden.

Eine Ausführungsform sieht vor, dass die Ermittlungseinheit dazu ausgebildet ist, den Messwert mit einem Referenzwert zu vergleichen und den Betriebszustand der Schleifkontakte basierend auf einem Ergebnis des Vergleichs zu ermitteln.

Eine Ausführungsform sieht vor, dass das Computertomographiegerät ferner eine Zeitreihen-Bereitstellungseinheit aufweist, welche zum Bereitstellen einer Zeitreihe des Messwerts und/oder eines Kennwerts, der basierend auf dem Messwert ermittelbar ist, ausgebildet ist, wobei die Ermittlungseinheit dazu ausgebildet ist, basierend auf der Zeitreihe eine Mustererkennung, insbesondere eine automatische Mustererkennung, durchzuführen und den Betriebszustand der Schleifkontakte basierend auf einem Ergebnis der Mustererkennung zu ermitteln. Dazu kann die Ermittlungseinheit einen oder mehrere entsprechend programmierte Prozessoren aufweisen.

Eine Ausführungsform sieht vor, dass die Mustererkennung basierend auf einem trainierten Maschinenlernalgorithmus durchgeführt wird.

Mittels der Messvorrichtung kann ein Messwert, welcher von der resultierenden Induktivität zwischen den zwei Schleifkontaktelementen abhängt, erfasst werden, insbesondere kontinuierlich erfasst werden. Der Messwert kann beispielsweise ein Wert für die resultierende Induktivität sein oder ein Wert für eine Resonanzfrequenz eines Schwingkreises sein, welcher die Induktivität des zumindest einen Abschnitts des Schleifbahn und die Induktivität des zumindest einen Abschnitts der Zuleitung aufweist. Zur Messung der resultierenden Induktivität kann jedes bekannte Verfahren angewendet werden.

Mittels der Ermittlungseinheit kann der Messwert insbesondere kontinuierlich mit einem Referenzwert verglichen werden. Bei dem Referenzwert kann es sich beispielsweise um einen Schwellwert handeln, dessen Abweichung von einem Normwert gerade noch tolerierbar ist. Wenn die Abweichung der resultierenden Induktivität von dem Normwert größer ist als die Abweichung des Referenzwerts von dem Normwert, kann beispielsweise als Betriebszustand der Schleifkontakte ein Zustand der Schleifkontakte ermittelt werden, der einen Austausch zumindest eines der zwei Schleifkontaktelemente und/oder eine Anpassung des Anpressdrucks zumindest eines der zwei Schleifkontaktelemente an die Schleifbahn erfordert.

Der Betriebszustand der Schleifkontakte kann basierend auf einem Ergebnis des Vergleichs beispielsweise ermittelt werden, indem zwischen einem Betriebszustand der Schleifkontakte, der keinen Austausch zumindest eines der zwei Schleifkontaktelemente erfordert, und einem Betriebszustand der Schleifkontakte, der den Austausch zumindest eines der zwei Schleifkontaktelemente erfordert, ausgewählt wird. Insbesondere kann der Betriebszustand beispielsweise mittels einer Anzeigevorrichtung angezeigt werden.

Zusätzlich zu der Zeitreihe des Messwerts und/oder des Kennwerts, der basierend auf dem Messwert ermittelt wird, kann eine weitere Zeitreihe mittels der Zeitreihen-Bereitstellungseinheit bereitgestellt werden, welche einen weiteren Kennwert betrifft, der vom Betriebszustand des Computertomographiegeräts abhängt und/oder der nicht von der resultierenden Induktivität zwischen den zwei Schleifkontaktelementen abhängt. Der weitere Kennwert kann beispielsweise die Rotationsgeschwindigkeit des Rotors des Computertomographiegeräts sein und/oder mit Hilfe von Sensoren ermittelt werden.

Die Mustererkennung kann beispielsweise die erste Ableitung des Messwerts nach der Zeit betreffen, insbesondere die Geschwindigkeit, mit der sich der Messwert zeitlich verändert. Die Mustererkennung kann insbesondere ferner basierend auf der weiteren Zeitreihe durchgeführt werden. Insbesondere kann die Mustererkennung einen Zusammenhang zwischen dem Messwert und dem weiteren Kennwert betreffen, beispielsweise einen Zusammenhang zwischen der resultierenden Induktivität und der Rotationsgeschwindigkeit.

Der Maschinenlernalgorithmus kann beispielsweise basierend auf einem Satz von Trainingspaaren trainiert werden. Insbesondere kann der Satz von Trainingspaaren bereitgestellt werden, wobei jedes Trainingspaar des Satzes von Trainingspaaren eine Trainingszeitreihe und einen Trainingsbetriebszustand, welcher der Trainingszeitreihe zugeordnet ist, aufweist.

Die Trainingspaare können beispielsweise ermittelt werden, indem ein zeitlicher Verlauf des Messwerts und/oder des Kennwerts beim Betrieb des Computertomographiegeräts gespeichert wird und für einen oder mehrere Zeitpunkte des zeitlichen Verlaufs jeweils ein Betriebszustand der Schleifkontakte als Trainingsbetriebszustand ermittelt und gespeichert. Ein Abschnitt des zeitlichen Verlaufs, welcher dem Zeitpunkt des Trainingsbetriebszustands unmittelbar vorangeht, wird als Trainingszeitreihe gespeichert. Dieser Trainingszeitreihe wird der Trainingsbetriebszustand zugeordnet, so dass ein Trainingspaar gebildet wird. Daten, beispielsweise die Zeitreihe und/oder der Satz von Trainingspaaren, können bereitgestellt werden, indem die Daten geladen werden, insbesondere aus einem Speichersystem und/oder in ein Prozessorsystems geladen werden.

Insbesondere kann zu einem Trainingsbetriebszustand, der fehlerhafte Schleifkontakte betrifft, ein Abschnitt des zeitlichen Verlaufs gespeichert werden, welcher dem Zeitpunkt, an dem der Fehler an den Schleifkontakten eingetreten ist, unmittelbar vorangeht und welcher sich ausreichend weit in die Vergangenheit erstreckt, so dass in der Trainingszeitreihe ein Muster, welches auf einen bevorstehenden Eintritt eines Fehlers an den Schleifkontakten hindeutet, erkannt werden kann.

Eine Ausführungsform sieht vor, dass die Messvorrichtung eine Kapazität aufweist, welche mit der resultierenden Induktivität zwischen den zwei Schleifkontaktelementen einen Parallelschwingkreis bildet,
- wobei der Messwert eine Resonanzfrequenz des Parallelschwingkreises ist.

Eine Ausführungsform sieht vor, dass die Messvorrichtung ein Koaxialkabel und eine Resonanzfrequenz-Ermittlungseinheit, welche zum Ermitteln der Resonanzfrequenz des Parallelschwingkreises ausgebildet ist, aufweist,
- wobei die zwei Schleifkontaktelemente mittels des Koaxialkabels derart mit der Resonanzfrequenz-Ermittlungseinheit verbunden sind, dass das Koaxialkabel zumindest einen Teil der Kapazität bildet, welche zusammen mit der resultierenden Induktivität zwischen den zwei Schleifkontaktelementen einen Parallelschwingkreis bildet.

Die Kapazität kann zumindest teilweise von dem Koaxialkabel und/oder zumindest teilweise von einem Kondensator gebildet sein. Eine Ausführungsform sieht vor, dass die Resonanzfrequenz-Ermittlungseinheit ein Netzwerkanalysator ist.

Eine Ausführungsform sieht vor, dass jedes der zwei Schleifkontaktelemente jeweils als eine Kohlebürste ausgebildet ist. Statt Kohlebürsten können auch andere bekannte Schleifkontaktelemente verwendet werden, beispielsweise Metallbürsten, insbesondere Bürsten aus Edelmetalldraht.

Die Erfindung betrifft ferner ein Verfahren zum Ermitteln eines Betriebszustands von Schleifkontakten an einem Schleifring für eine Drehübertragung, insbesondere für eine Drehübertragung von elektrischer Energie zur Versorgung einer Röntgenquelle, in einem Computertomographiegerät, wobei das Verfahren die folgenden Schritte umfasst:
- Erfassen eines Messwerts, welche von der resultierenden Induktivität zwischen zwei Schleifkontaktelementen abhängt, welche die Schleifkontakte zwischen eines Schleifbahn des Schleifrings und einer Zuleitung bilden und welche derart zueinander parallel geschaltet sind, dass die zwei Schleifkontaktelemente mittels zumindest eines Abschnitts der Schleifbahn und mittels zumindest eines Abschnitts der Zuleitung miteinander elektrisch leitend verbunden sind, wobei der Schleifring relativ zu der Zuleitung drehbar angeordnet ist,
- Ermitteln des Betriebszustands der Schleifkontakte basierend auf dem Messwert.

Eine Ausführungsform sieht vor, dass der Messwert mit einem Referenzwert verglichen wird, wobei der Betriebszustand der Schleifkontakte basierend auf einem Ergebnis des Vergleichs ermittelt wird.

Eine Ausführungsform sieht vor, dass eine Zeitreihe des Messwerts und/oder eines Kennwerts, der basierend auf dem Messwert ermittelt wird, bereitgestellt wird,
- wobei basierend auf der Zeitreihe eine Mustererkennung, insbesondere eine automatische Mustererkennung, durchgeführt wird,
- wobei der Betriebszustand der Schleifkontakte basierend auf einem Ergebnis der Mustererkennung ermittelt wird.

Eine Ausführungsform sieht vor, dass die Mustererkennung basierend auf einem trainierten Maschinenlernalgorithmus durchgeführt wird.

Eine Ausführungsform sieht vor, dass ein Parallelschwingkreis aus einer Kapazität und der resultierenden Induktivität zwischen den zwei Schleifkontaktelementen gebildet wird, - wobei der Messwert eine Resonanzfrequenz des Parallelschwingkreises ist.

Eine Ausführungsform sieht vor, dass in Abhängigkeit von dem Betriebszustand der Schleifkontakte zumindest eines der zwei Schleifkontaktelemente ausgetauscht wird und/oder ein Signal, welches darauf hinweist, dass ein Austausch zumindest eines der zwei Schleifkontaktelemente erforderlich ist, ausgegeben wird. Dieses Signal kann beispielsweise in einem entfernten Service-Zentrum ausgegeben werden, welches sich insbesondere außerhalb des Krankenhauses befindet, in dem das Computertomographiegerät betrieben wird.

Eine Ausführungsform sieht vor, dass in Abhängigkeit von dem Betriebszustand der Schleifkontakte ein Anpressdruck zumindest eines der zwei Schleifkontaktelemente an die Schleifbahn verändert, insbesondere erhöht, wird.

Eine Ausführungsform sieht vor, dass der Messwert erfasst wird, während mittels der zwei Schleifkontaktelemente elektrische Energie zur Versorgung einer Röntgenquelle von der Zuleitung auf die Schleifbahn übertragen wird.

Eine Ausführungsform sieht vor, dass die elektrische Energie zur Versorgung der Röntgenquelle in Form zumindest eines hochfrequenten Pulsstroms übertragen wird. Die elektrische Energie kann in der Röntgenquelle zumindest teilweise in Röntgenstrahlung umgewandelt werden.

Die Ermittlung des Betriebszustandes der Schleifkontakte ist somit weitgehend unabhängig vom aktuellen Spannungs- und Stromverlauf an den Schleifkontaktelementen und weitgehend unabhängig von der Position des Schleifkontakts entlang der Schleifbahn, insbesondere weitgehend unabhängig davon, ob sich im Bereich des Schleifkontakts ein Nulldurchgang des Stroms oder der Spannung, mit der die elektrische Energie übertragen wird, befindet. Damit erfolgt die Ermittlung des Betriebszustandes der Schleifkontakte weitgehend unbeeinträchtigt durch Störungen, die während einer Übertragung von elektrischer Energie zum Betrieb einer Röntgenröhre auftreten. Bei solchen Störungen kann es sich beispielsweise um Oberwellen von Wechselrichtern handeln.

Ferner kann auf diese Weise der Betriebszustand von Schleifkontakten, die zur Drehübertragung eines Schutzleiters vorgesehen sind, ermittelt werden. Insbesondere ist es dafür nicht erforderlich, einen Teststrom in den Schutzleiter einzuspeisen.

Der Betriebszustand der Schleifkontakte kann daher insbesondere auch während eines Akquisitionsvorgangs ermittelt werden, bei dem Röntgenstrahlung mittels der Röntgenröhre erzeugt und nach einer Wechselwirkung mit einem abzubildenden Bereich des Patienten mittels eines Röntgendetektors erfasst wird.

In Abhängigkeit von dem Betriebszustand der Schleifkontakte kann zumindest eines der zwei Schleifkontaktelemente ausgetauscht werden und/oder ein Zeitpunkt oder ein Zeitintervall für den Austausch des zumindest einen der zwei Schleifkontaktelemente ermittelt werden. Dadurch, dass die Überwachung des Betriebszustands der Schleifkontaktelemente kontinuierlich möglich ist, kann die Lebensdauer der Schleifkontaktelemente weitgehend ausgenutzt werden und eine mögliche Beschädigung der Schleifbahn durch fehlerhafte Schleifkontakte weitgehend vermieden werden. Dadurch können die Wartungskosten verringert, durch fehlerhafte Schleifkontakte bedingte Abbrüche von Untersuchungen vermieden und Ausfallzeiten des Computertomographiegeräts besser geplant werden.

Unter einem Maschinenlernalgorithmus wird im Kontext dieser Anmeldung insbesondere ein Algorithmus, der zum Maschinellen Lernen ausgebildet ist, verstanden. Ein Maschinenlernalgorithmus kann beispielsweise mit Hilfe von Entscheidungsbäumen, mathematischen Funktionen und/oder allgemeinen Programmiersprachen realisiert werden. Der Maschinenlernalgorithmus kann beispielsweise zum überwachten Lernen und/oder zum unüberwachten Lernen ausgebildet sein. Der Maschinenlernalgorithmus kann beispielsweise zum tiefen Lernen ("deep learning") und/oder zum bestärkendem Lernen ("reinforcement learning") und/oder zum Marginal Space Learning ausgebildet sein. Insbesondere beim überwachten Lernen kann eine Funktionenklasse verwendet werden, welche beispielsweise auf Entscheidungsbäumen ("decision trees"), einem Random Forest, einer logistischen Regression, einer Support Vector Machine, einem künstlichen neuronalen Netz, einer Kernel-Methode, Bayes-Klassifikatoren oder ähnlichem oder Kombinationen davon basiert. Mögliche Implementierungen des Maschinenlernalgorithmus können beispielsweise künstliche Intelligenz verwenden. Berechnungen, insbesondere beim Trainieren eines Maschinenlernalgorithmus, können beispielsweise mittels eines Prozessorsystems ausgeführt werden. Das Prozessorsystem kann beispielsweise einen oder mehrere Prozessoren, insbesondere Grafikprozessoren, aufweisen.

Im Rahmen der Erfindung können Merkmale, welche in Bezug auf unterschiedliche Ausführungsformen der Erfindung und/oder unterschiedliche Anspruchskategorien (Verfahren, Verwendung, Vorrichtung, System, Anordnung usw.) beschrieben sind, zu weiteren Ausführungsformen der Erfindung kombiniert werden. Beispielsweise kann ein Anspruch, der eine Vorrichtung betrifft, auch mit Merkmalen, die im Zusammenhang mit einem Verfahren beschrieben oder beansprucht sind, weitergebildet werden und umgekehrt. Funktionale Merkmale eines Verfahrens können dabei durch entsprechend ausgebildete gegenständliche Komponenten ausgeführt werden. Neben den in dieser Anmeldung ausdrücklich beschriebenen Ausführungsformen der Erfindung sind vielfältige weitere Ausführungsformen der Erfindung denkbar, zu denen der Fachmann gelangen kann, ohne den Bereich der Erfindung zu verlassen, soweit er durch die Ansprüche vorgegeben ist.

Die Verwendung der unbestimmten Artikel "ein" bzw. "eine" schließt nicht aus, dass das betroffene Merkmal auch mehrfach vorhanden sein kann. Die Verwendung des Zahlworts "zwei" schließt nicht aus, dass das betroffene Merkmal auch mehr als zweimal vorhanden sein kann. Die Verwendung des Ausdrucks "aufweisen" schließt nicht aus, dass die mittels des Ausdrucks "aufweisen" verknüpften Begriffe identisch sein können. Beispielsweise weist die medizinische Bildgebungsvorrichtung die medizinische Bildgebungsvorrichtung auf. Die Verwendung des Ausdrucks "Einheit" schließt nicht aus, dass der Gegenstand, auf den sich der Ausdruck "Einheit" bezieht, mehrere Komponenten aufweisen kann, die räumlich voneinander separiert sind.

Der Ausdruck "basierend auf" kann im Kontext der vorliegenden Anmeldung insbesondere im Sinne des Ausdrucks "unter Verwendung von" verstanden werden. Insbesondere schließt eine Formulierung, der zufolge ein erstes Merkmal basierend auf einem zweiten Merkmal erzeugt (alternativ: ermittelt, bestimmt etc.) wird, nicht aus, dass das erste Merkmal basierend auf einem dritten Merkmal erzeugt (alternativ: ermittelt, bestimmt etc.) werden kann.

Im Folgenden wird die Erfindung anhand von Ausführungsbeispielen unter Hinweis auf die beigefügten Figuren erläutert. Die Darstellung in den Figuren ist schematisch, stark vereinfacht und nicht zwingend maßstabsgetreu.

Es zeigen:
die Fig. 1 ein Ausführungsbeispiel mit einem Schleifring, einer Zuleitung und zwei Schleifkontaktelementen,
die Fig. 2 ein Ausführungsbeispiel mit einem Schleifring, einer Zuleitung und zwei Schleifkontaktelementen in Verbindung mit einer Kapazität,
die Fig. 3 zeigt ein Ausführungsbeispiel mit einem Schleifring, der mehrere Schleifbahnen aufweist, und mehreren Zuleitungen,
die Fig. 4 einen Schaltplan mit einem Parallelschwingkreis,
die Fig. 5 ein Ausführungsbeispiel mit einer Zuleitung, welche zwei Leiterbahnabschnitte aufweist,
die Fig. 6 ein Diagramm für die Impedanz des Parallelschwingkreises,
die Fig. 7 ein Diagramm für die Impedanz des Parallelschwingkreises, wobei ein Schleifkontakt unterbrochen ist,
die Fig. 8 ein Computertomographiegerät und
die Fig. 9 ein Ablaufdiagramm für ein Verfahren zum Ermitteln eines Betriebszustands von Schleifkontakten.

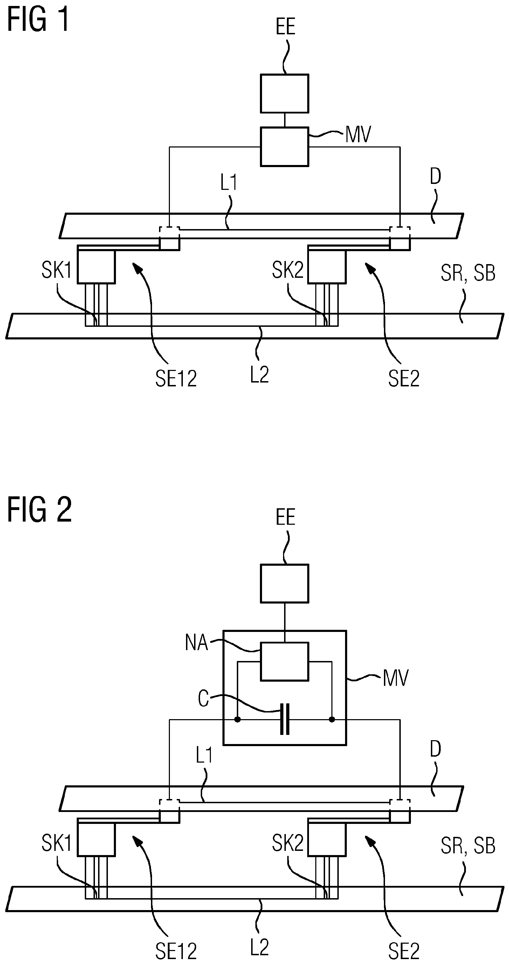

Die Fig. 1 zeigt den Schleifring SR mit der Schleifbahn SB, die Zuleitung D, die Schleifkontaktelemente SE1 und SE2, die Schleifkontakte SK1 und SK2, die Messvorrichtung MV und die Ermittlungseinheit EE. Der Schleifring SR weist die Schleifbahn SB auf und ist relativ zu der Zuleitung D drehbar angeordnet. An der Zuleitung D sind die zwei Schleifkontaktelemente SE1 und SE2 angeordnet. Jedes der zwei Schleifkontaktelemente SE1 und SE2 ist jeweils als eine Bürste, beispielsweise als eine Kohlebürste, ausgebildet.

Jedes der zwei Schleifkontaktelemente SE1 und SE2 ist jeweils mittels eines Federelements und einer Anpressdruck-Einstellvorrichtung, die zum Einstellen des Anpressdrucks der Bürste an die Schleifbahn SB ausgebildet ist, mit der Zuleitung D verbunden. Ein Ende des Federelements ist fest mit der Zuleitung D verbunden. Das andere Ende des Federelements ist fest mit der Bürste verbunden. Das Federelement kann beispielsweise einen elastischen Metallstreifen aufweisen, welches einseitig im Bereich der Zuleitung D eingespannt ist. Die Anpressdruck-Einstellvorrichtung kann beispielsweise ein Gewinde aufweisen und/oder dazu ausgebildet sein, den Abstand der Bürste relativ zu der Zuleitung D zu verändern. Durch Erhöhen des Abstandes der Bürste relativ zu der Zuleitung D kann der Anpressdruck der Bürste an die Schleifbahn SB erhöht werden. Bei einer Bürste, die durch Verschleiß abgenutzt ist, kann auf diese Weise ein funktionierender Schleifkontakt wiederhergestellt werden.

Die zwei Schleifkontaktelemente SE1 und SE2 sind derart zueinander parallel geschaltet, dass die zwei Schleifkontaktelemente SE1 und SE2 mittels des Abschnitts L2 der Schleifbahn SB und mittels des Abschnitts L1 der Zuleitung D miteinander elektrisch leitend verbunden sind. Die zwei Schleifkontaktelemente SE1 und SE2 sind an demselben Leiterbahnabschnitt der Zuleitung D angeordnet. Die Schleifbahn SB ist sowohl mittels des Schleifkontaktelements SE1 als auch mittels des Schleifkontaktelements SE2 mit der Zuleitung D elektrisch leitend verbunden. Das Schleifkontaktelement SE1 ist eine erste Kohlebürste. Das Schleifkontaktelement SE2 ist eine zweite Kohlebürste.

Es können noch ein oder mehrere weitere, in der Fig. 1 nicht dargestellte Schleifkontaktelemente vorgesehen sind, welche jeweils einen Schleifkontakt zwischen der Schleifbahn und der Zuleitung bilden. Statt einer Leiterbahn kann für die Zuleitung D beispielsweise Litze verwendet werden, um die zwei Schleifkontaktelemente SE1 und SE2 miteinander elektrisch leitend zu verbinden. Insbesondere können mehrere Leiterbahnabschnitte vorgesehen sein. Insbesondere kann für jede Phase einer mehrphasigen elektrischen Energieübertragung jeweils ein Leiterbahnabschnitt und jeweils eine Schleifbahn des Schleifrings SR vorgesehen sein.

Die resultierende Induktivität zwischen den zwei Schleifkontaktelementen SE1 und SE2 hängt insbesondere von der Induktivität des Abschnitts L1 der Zuleitung D, von der Induktivität des Abschnitts L2 der Schleifbahn SB und von dem Betriebszustand der Schleifkontakte SK1 und SK2 ab. Wenn die Schleifkontakte SK1 und SK2 optimal ausgebildet sind, entspricht die resultierende Induktivität im Wesentlichen der Parallelschaltung der Induktivität des Abschnitts L1 und der Induktivität des Abschnitts L2. Ist zumindest einer der Schleifkontakte SK1 und SK2 unterbrochen, entspricht die resultierende Induktivität im Wesentlichen der Induktivität des Abschnitts L1.

Die Fig. 2 zeigt den Schleifring SR mit der Schleifbahn SB, die Zuleitung D, die Schleifkontaktelemente SE1 und SE2, die Schleifkontakte SK1 und SK2, die Messvorrichtung MV und die Ermittlungseinheit EE. Die Messvorrichtung MV weist eine Kapazität C auf, welche mit der resultierenden Induktivität zwischen den zwei Schleifkontaktelementen SE1 und SE2 einen Parallelschwingkreis bildet. Die zwei Schleifkontaktelementen SE1 und SE2 sind mittels der Kapazität C miteinander verbunden und die Kapazität C ist parallel zu dem Abschnitt L1 der Zuleitung geschaltet. Die Messvorrichtung MV weist ferner einen vektoriellen Netzwerkanalysator NA als Resonanzfrequenz-Ermittlungseinheit auf, mit dem die Resonanzfrequenz des Parallelschwingkreises ermittelt werden kann.

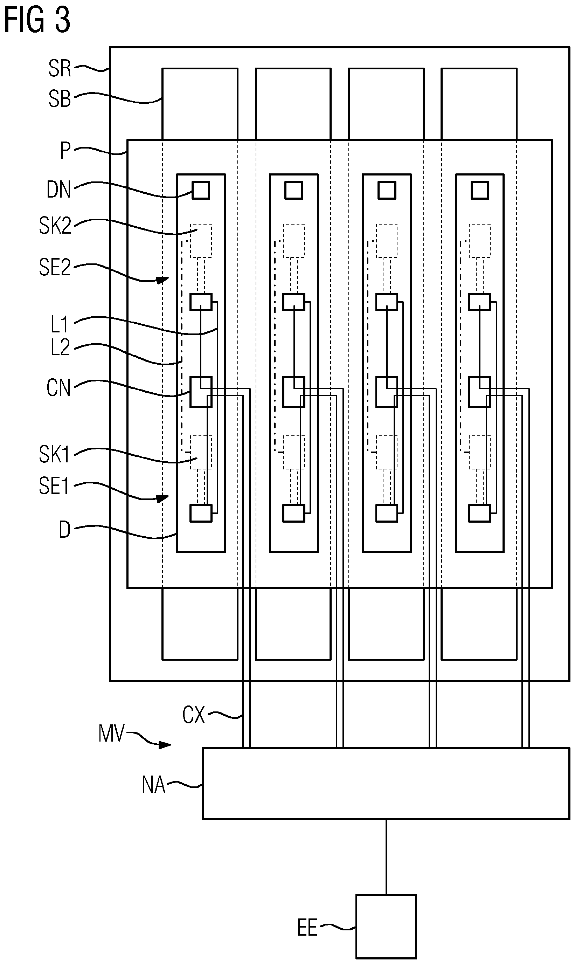

Die Fig. 3 zeigt den Schleifring SR, der mehrere Schleifbahnen aufweist, und die Platine P, die mehrere Zuleitungen in Form von Leiterbahnen aufweist. Der Schleifring SR ist relativ zu der Platine P drehbar angeordnet. Drei Zuleitungen sind für die drei Phasen einer dreiphasigen elektrischen Energieübertragung vorgesehen. Eine vierte Zuleitung ist zusammen mit der entsprechenden Schleifbahn für die Drehübertragung des Schutzleiters vorgesehen. An jeder der vier Zuleitungen sind jeweils zwei Schleifkontaktelemente angeordnet, welche mittels eines von vier Koaxialkabeln, welches der jeweiligen Zuleitung zugeordnet ist, mit einer Resonanzfrequenz-Ermittlungseinheit verbunden ist.

Für jede der vier Zuleitungen wird jeweils eine Resonanzfrequenz, welcher von der resultierenden Induktivität zwischen den zwei Schleifkontaktelementen der Zuleitung abhängt, erfasst. Die Resonanzfrequenz-Ermittlungseinheit kann insbesondere dazu ausgebildet sein, die vier Resonanzfrequenzen zeitlich simultan zu ermitteln. Dazu kann die Resonanzfrequenz-Ermittlungseinheit beispielsweise einen vierkanaligen Netzwerkanalysator NA oder vier einkanalige Netzwerkanalysatoren aufweisen. Alternativ kann die Resonanzfrequenz-Ermittlungseinheit dazu ausgebildet sein, zwischen den vier Koaxialkabeln derart umzuschalten, dass die vier Resonanzfrequenzen zeitlich aufeinanderfolgend und wiederholt ermittelt werden. Somit wäre ein einkanaliger Netzwerkanalysator ausreichend, um alle vier Resonanzfrequenzen zu ermitteln.

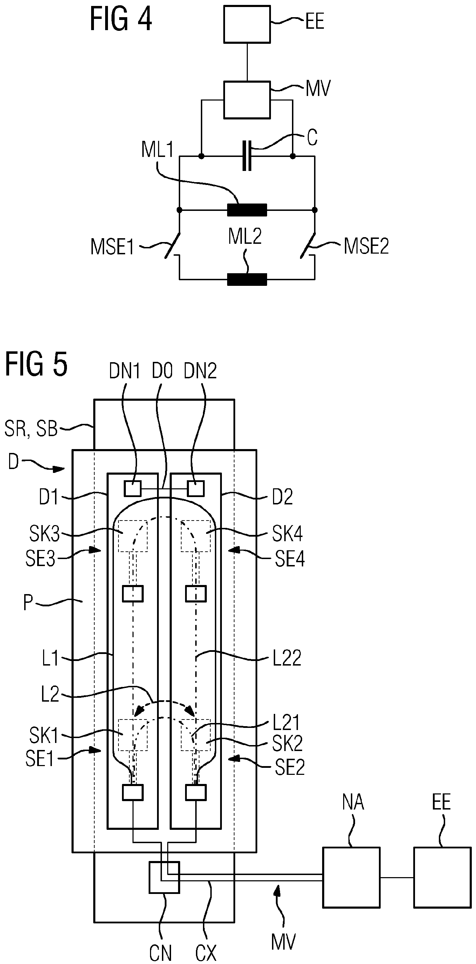

Die Fig. 4 zeigt einen Schaltplan mit einem Parallelschwingkreis, der Messvorrichtung MV und der Ermittlungseinheit EE. Der Parallelschwingkreis weist die Kapazität C und die Induktivität ML1 auf. Wenn beide Schalter MSE1 und MSE2 geschlossen sind, weist der Parallelschwingkreis ferner die Induktivität ML2 auf, so dass die resultierende Induktivität des Parallelschwingkreises einer Parallelschaltung der Induktivität ML1 und ML2 entspricht. Die Induktivität ML1 entspricht im Wesentlichen der Induktivität des Abschnitts L1 der Zuleitung D. Die Induktivität ML2 entspricht im Wesentlichen der Induktivität des Abschnitts L2 der Schleifbahn SB.

Der Schalter MSE1 entspricht dem Schleifkontaktelement SE1. Der Schalter MSE2 entspricht dem Schleifkontaktelement SE2. Ein geschlossener Schalter entspricht einem intakten Schleifkontakt zwischen der Schleifbahn SB und der Zuleitung D. Ein offener Schalter entspricht einem fehlerhaften, insbesondere unterbrochenen, Schleifkontakt zwischen der Schleifbahn SB und der Zuleitung D.

In dem in Fig. 5 gezeigten Beispiel weist die Zuleitung D einen ersten Leiterbahnabschnitt D1, einen zweiten Leiterbahnabschnitt D2, der parallel zu dem ersten Leiterbahnabschnitt D1 angeordnet ist und eine Verbindungsleitung D0, welche den zweiten Leiterbahnabschnitt D2 mit dem ersten Leiterbahnabschnitt D1 elektrisch leitend verbindet, auf. Die Verbindungsleitung D0 kann beispielsweise eine Litze sein. Die Verbindungsleitung D0 ist mit dem Anschluss DN1 des ersten Leiterbahnabschnitts D1 und dem Anschluss DN2 des zweiten Leiterbahnabschnitts D2 verbunden.

Auf diese Weise kann ein Abschnitt L1 der Zuleitung realisiert werden, welcher eine relativ große Länge und damit eine relativ große Induktivität aufweist. Damit kann ein Parallelschwingkreis ausgebildet werden, dessen Resonanzfrequenz noch sensitiver von der Induktivität des Abschnitts L2 der Schleifbahn abhängt. Die Induktivität des Abschnitts L2 der Schleifbahn SB resultiert aus den Induktivitäten mehrerer Pfade, welche die zwei Schleifkontaktelemente SE1 und SE2 miteinander mehrerer elektrisch leitend verbinden. Insbesondere sind die zwei Schleifkontaktelemente SE1 und SE2 mittels der Pfade L21 und L22 miteinander elektrisch leitend verbunden.

Die zwei Schleifkontaktelemente SE1 und SE3 sind am ersten Leiterbahnabschnitt D1 der Zuleitung D angeordnet. Die zwei Schleifkontaktelemente SE2 und SE4 sind am zweiten Leiterbahnabschnitt D1 der Zuleitung D angeordnet. Das Schleifkontaktelement SE1 bildet den Schleifkontakt SK1 zwischen der Schleifbahn SB und dem ersten Leiterabschnitt D1 der Zuleitung D. Das Schleifkontaktelement SE3 bildet den Schleifkontakt SK3 zwischen der Schleifbahn SB und dem ersten Leiterabschnitt D1 der Zuleitung D. Das Schleifkontaktelement SE2 bildet den Schleifkontakt SK2 zwischen der Schleifbahn SB und dem zweiten Leiterabschnitt D2 der Zuleitung D. Das Schleifkontaktelement SE4 bildet den Schleifkontakt SK4 zwischen der Schleifbahn SB und dem zweiten Leiterabschnitt D2 der Zuleitung D.

Die Messvorrichtung MV weist einen vektoriellen Netzwerkanalysator NA als Resonanzfrequenz-Ermittlungseinheit auf. Der vektorielle Netzwerkanalysator NA ist mittels des Koaxialkabels CX und der Steckverbindung CN, beispielsweise in Form einer SMA-Verbindung, mit den zwei Schleifkontaktelementen SE1 und SE2 verbunden. Die Kapazität des Koaxialkabels CX bildet mit der resultierenden Induktivität zwischen den zwei Schleifkontaktelementen SE1 und SE2 einen Parallelschwingkreis.

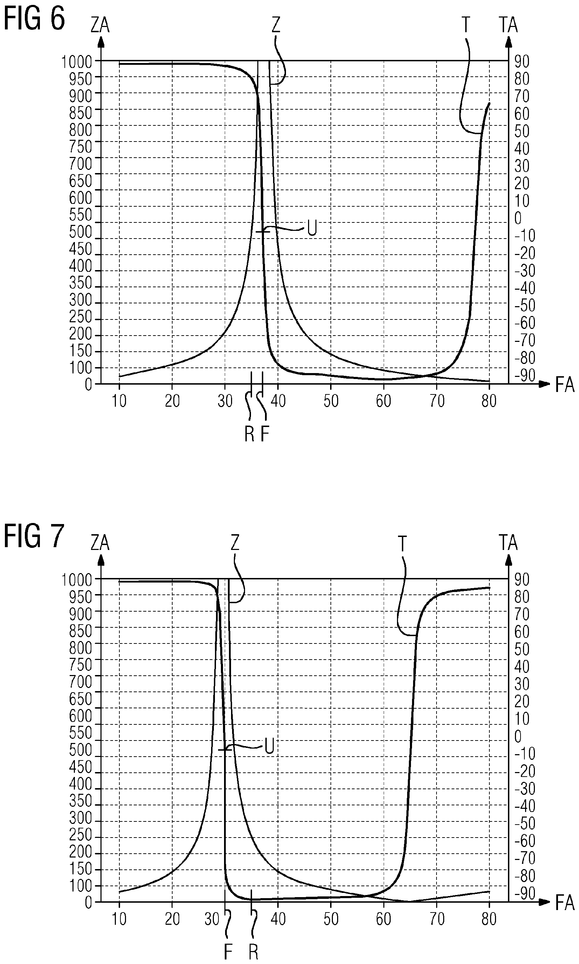

Die Fig. 6 zeigt ein Diagramm , in dem der Betrag Z und der Phasenwinkel T der Impedanz des Parallelschwingkreises in Abhängigkeit von der Frequenz aufgetragen sind, wobei die Schleifkontaktelemente SE1 und SE2 mit einem grenzwertig geringen Anpressdruck an der Schleifbahn SB anliegen und die Schleifkontakte SK1 und SK2 bilden.
Auf der Achse ZA sind Werte in Ohm für den Betrag Z der Impedanz des Parallelschwingkreises eingetragen. Auf der Achse TA sind Werte in Grad für den Phasenwinkel T der Impedanz des Parallelschwingkreises eingetragen. Auf der Achse FA sind Werte in Megahertz für die Frequenz eingetragen. Der Marker U befindet sich auf der Resonanzfrequenz F von ca. 37 Megahertz, welche größer ist als der Referenzwert R von ca. 35 Megahertz.

Die Fig. 7 zeigt das Diagramm, wobei eines der Schleifkontaktelemente SE1 und SE2 mittels einer dünnen Folie von der Schleifbahn SB isoliert wurde, so dass der entsprechende Schleifkontakt unterbrochen ist. Der Marker U befindet sich auf der Resonanzfrequenz F von ca. 30 Megahertz, welche kleiner ist als der Referenzwert R von ca. 35 Megahertz.

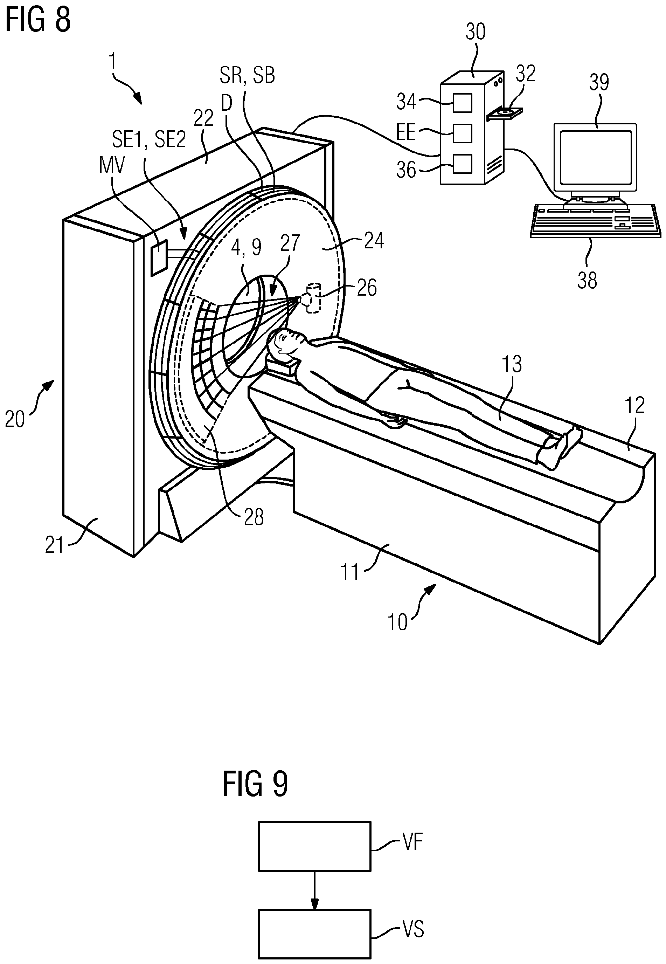

Die Fig. 8 zeigt das Computertomographiegerät 1, welches die Gantry 20, die tunnelförmige Öffnung 9, die Patientenlagerungsvorrichtung 10 und die Steuerungsvorrichtung 30 aufweist. Die Gantry 20 weist den Tragrahmen 21, den Kipprahmen 22 und den Rotor 24 auf. Der Kipprahmen 22 ist mittels einer Kipplagerungsvorrichtung an dem Tragrahmen 21 um eine Kippachse relativ zu dem Tragrahmen 21 kippbar angeordnet. Der Rotor 24 ist mittels einer Drehlagerungsvorrichtung an dem Kipprahmen 22 um eine Rotationsachse relativ zu dem Kipprahmen 22 drehbar angeordnet. Die Kippachse ist horizontal und senkrecht zu der Rotationsachse.

Das Computertomographiegerät 1 weist den Schleifring SR mit der Schleifbahn SB, die Zuleitung D, die zwei Schleifkontaktelemente SE1 und SE2, welche die Schleifkontakte SK1 und SK2 zwischen der Schleifbahn SB des Schleifrings SR und der Zuleitung D bilden, die Messvorrichtung MV und die Ermittlungseinheit EE auf. Die Zuleitung D ist an dem Kipprahmen 22 relativ zu dem Kipprahmen 22 feststehend angeordnet. Der Schleifring SR ist an dem Rotor 24 relativ zu dem Rotor 24 feststehend angeordnet. Somit ist der Schleifring SR relativ zu der Zuleitung D drehbar, insbesondere um die Rotationsachse des Rotors 24 drehbar, angeordnet.

In die tunnelförmige Öffnung 9 ist der Patient 13 einführbar. In der tunnelförmigen Öffnung 9 befindet sich der Akquisitionsbereich 4. In dem Akquisitionsbereich 4 ist ein abzubildender Bereich des Patienten 13 derart positionierbar, dass die Strahlung 27 von der Strahlungsquelle 26 zu dem abzubildenden Bereich gelangen kann und nach einer Wechselwirkung mit dem abzubildenden Bereich zu dem Strahlungsdetektor 28 gelangen kann.

Die Patientenlagerungsvorrichtung 10 weist den Lagerungssockel 11 und die Lagerungsplatte 12 zur Lagerung des Patienten 13 auf. Die Lagerungsplatte 12 ist derart relativ zu dem Lagerungssockel 11 bewegbar an dem Lagerungssockel 11 angeordnet, dass die Lagerungsplatte 12 in einer Längsrichtung der Lagerungsplatte 12 in den Akquisitionsbereich 4 einführbar ist.

Das Computertomographiegerät 1 ist zur Akquisition von Akquisitionsdaten basierend auf einer elektromagnetischen Strahlung 27 ausgebildet und weist eine Akquisitionseinheit auf. Die Akquisitionseinheit ist eine Projektionsdaten-Akquisitionseinheit mit der Strahlungsquelle 26, z. B. einer Röntgenquelle, insbesondere einer Röntgenröhre, und dem Detektor 28, z. B. einem Röntgendetektor, insbesondere einem energieauflösenden Röntgendetektor.

Die Strahlungsquelle 26 ist an dem Rotor 24 angeordnet und zur Emission einer Strahlung 27, z. B. einer Röntgenstrahlung, mit Strahlungsquanten 27 ausgebildet. Der Detektor 28 ist an dem Rotor 24 angeordnet und zur Detektion der Strahlungsquanten 27 ausgebildet. Die Strahlungsquanten 27 können von der Strahlungsquelle 26 zu dem abzubildenden Bereich des Patienten 13 gelangen und nach einer Wechselwirkung mit dem abzubildenden Bereich auf den Detektor 28 auftreffen. Auf diese Weise können mittels der Akquisitionseinheit Akquisitionsdaten des abzubildenden Bereichs in Form von Projektionsdaten erfasst werden.

Die Steuerungsvorrichtung 30 ist zum Empfangen der von der Akquisitionseinheit akquirierten Akquisitionsdaten ausgebildet. Die Steuerungsvorrichtung 30 ist zum Steuern des Computertomographiegeräts 1 ausgebildet.

Die Steuerungsvorrichtung 30 weist die Ermittlungseinheit EE, das computerlesbare Medium 32 und das Prozessorsystem 36 auf. Die Steuerungsvorrichtung 30, insbesondere die Ermittlungseinheit EE, wird von einem Datenverarbeitungssystem, welches einen Computer aufweist, gebildet. Die Steuerungsvorrichtung 30 weist die Bildrekonstruktionseinrichtung 34 auf. Mittels der Bildrekonstruktionseinrichtung 34 kann basierend auf den Akquisitionsdaten ein medizinischer Bilddatensatz rekonstruiert werden.

Die medizinische Bildgebungsvorrichtung 1 weist eine Eingabevorrichtung 38 und eine Ausgabevorrichtung 39 auf, welche jeweils mit der Steuerungsvorrichtung 30 verbunden sind. Die Eingabevorrichtung 38 ist zum Eingeben von Steuerungs-Informationen, z. B. Bildrekonstruktionsparametern, Untersuchungsparametern oder ähnliches, ausgebildet. Die Ausgabevorrichtung 39 ist insbesondere zum Ausgeben von Steuerungs-Informationen, Bildern und/oder akustischen Signalen ausgebildet. Insbesondere kann der Betriebszustand der Schleifkontakte SK1 und SK2 mittels der Ausgabevorrichtung 39 angezeigt werden.

Die Fig. 9 zeigt ein Ablaufdiagramm für ein Verfahren zum Ermitteln des Betriebszustands der Schleifkontakte SK1 und SK2 an dem Schleifring SR für eine Drehübertragung in dem Computertomographiegerät 1, wobei das Verfahren die folgenden Schritte umfasst:
- Erfassen VF eines Messwerts F, welche von der resultierenden Induktivität zwischen zwei Schleifkontaktelementen SE1, SE2 abhängt, welche die Schleifkontakte SK1, SK2 zwischen eines Schleifbahn SB des Schleifrings SR und einer Zuleitung D bilden und welche derart zueinander parallel geschaltet sind, dass die zwei Schleifkontaktelemente SE1 und SE2 mittels zumindest eines Abschnitts L2 der Schleifbahn SB und mittels zumindest eines Abschnitts L1 der Zuleitung D miteinander elektrisch leitend verbunden sind, wobei der Schleifring SR relativ zu der Zuleitung D drehbar angeordnet ist,
- Ermitteln VS des Betriebszustands der Schleifkontakte SK1 und SK2 basierend auf dem Messwert F.

## Patentansprüche

1. Computertomographiegerät (1), aufweisend
- einen Schleifring (SR) für eine Drehübertragung,
- eine Zuleitung (D), wobei der Schleifring (SR) relativ zu der Zuleitung (D) drehbar angeordnet ist,
- zwei Schleifkontaktelemente (SE1, SE2), welche Schleifkontakte (SK1, SK2) zwischen einer Schleifbahn (SB) des Schleifrings (SR) und der Zuleitung (D) bilden und welche derart zueinander parallel geschaltet sind, dass die zwei Schleifkontaktelemente (SE1, SE2) mittels zumindest eines Abschnitts (L2) der Schleifbahn (SB) und mittels zumindest eines Abschnitts (L1) der Zuleitung (D) miteinander elektrisch leitend verbunden sind,
- eine Messvorrichtung (MV), welche zum Erfassen eines Messwerts (F), welcher von der resultierenden Induktivität zwischen den zwei Schleifkontaktelementen (SE1, SE2) abhängt, ausgebildet ist,
- eine Ermittlungseinheit (EE), welche zum Ermitteln eines Betriebszustands der Schleifkontakte (SK1, SK2) basierend auf dem Messwert (F) ausgebildet ist.

2. Computertomographiegerät (1) nach Anspruch 1,
- wobei die Ermittlungseinheit (EE) dazu ausgebildet ist, den Messwert (F) mit einem Referenzwert (R) zu vergleichen und den Betriebszustand der Schleifkontakte (SK1, SK2) basierend auf einem Ergebnis des Vergleichs zu ermitteln.

3. Computertomographiegerät (1) nach Anspruch 1 oder 2,
- wobei die Messvorrichtung (MV) eine Kapazität aufweist, welche mit der resultierenden Induktivität zwischen den zwei Schleifkontaktelementen (SE1, SE2) einen Parallelschwingkreis bildet,
- wobei der Messwert (F) eine Resonanzfrequenz des Parallelschwingkreises ist.

4. Computertomographiegerät (1) nach Anspruch 3,
- wobei die Messvorrichtung (MV) ein Koaxialkabel (CX) und eine Resonanzfrequenz-Ermittlungseinheit, welche zum Ermitteln der Resonanzfrequenz des Parallelschwingkreises ausgebildet ist, aufweist,
- wobei die zwei Schleifkontaktelemente (SE1, SE2) mittels des Koaxialkabels (CX) derart mit der Resonanzfrequenz-Ermittlungseinheit verbunden sind, dass das Koaxialkabel (CX) zumindest einen Teil der Kapazität bildet, welche zusammen mit der resultierenden Induktivität zwischen den zwei Schleifkontaktelementen (SE1, SE2) einen Parallelschwingkreis bildet.

5. Computertomographiegerät (1) nach Anspruch 4,
- wobei die Resonanzfrequenz-Ermittlungseinheit ein Netzwerkanalysator (NA) ist.

6. Computertomographiegerät (1) nach einem der Ansprüche 1 bis 5,
- wobei jedes der zwei Schleifkontaktelemente (SE1, SE2) jeweils als eine Kohlebürste ausgebildet ist.

7. Verfahren zum Ermitteln eines Betriebszustands von Schleifkontakten (SK1, SK2) an einem Schleifring (SR) für eine Drehübertragung in einem Computertomographiegerät (1), wobei das Verfahren die folgenden Schritte umfasst:
- Erfassen (VF) eines Messwerts (F), welche von der resultierenden Induktivität zwischen zwei Schleifkontaktelementen (SE1, SE2) abhängt, welche die Schleifkontakte (SK1, SK2) zwischen eines Schleifbahn (SB) des Schleifrings (SR) und einer Zuleitung (D) bilden und welche derart zueinander parallel geschaltet sind, dass die zwei Schleifkontaktelemente (SE1, SE2) mittels zumindest eines Abschnitts (L2) der Schleifbahn (SB) und mittels zumindest eines Abschnitts (L1) der Zuleitung (D) miteinander elektrisch leitend verbunden sind, wobei der Schleifring (SR) relativ zu der Zuleitung (D) drehbar angeordnet ist,
- Ermitteln (VS) des Betriebszustands der Schleifkontakte (SK1, SK2) basierend auf dem Messwert (F).

8. Verfahren nach Anspruch 7,
- wobei der Messwert (F) mit einem Referenzwert (R) verglichen wird
- wobei der Betriebszustand der Schleifkontakte (SK1, SK2) basierend auf einem Ergebnis des Vergleichs ermittelt wird.

9. Verfahren nach Anspruch 7 oder 8,
- wobei eine Zeitreihe des Messwerts (F) und/oder eines Kennwerts, der basierend auf dem Messwert (F) ermittelt wird, bereitgestellt wird,
- wobei basierend auf der Zeitreihe eine Mustererkennung durchgeführt wird,
- wobei der Betriebszustand der Schleifkontakte (SK1, SK2) basierend auf einem Ergebnis der Mustererkennung ermittelt wird.

10. Verfahren nach Anspruch 9,
- wobei die Mustererkennung basierend auf einem trainierten Maschinenlernalgorithmus durchgeführt wird.

11. Verfahren nach einem der Ansprüche 7 bis 10,
- wobei ein Parallelschwingkreis aus einer Kapazität und der resultierenden Induktivität zwischen den zwei Schleifkontaktelementen (SE1, SE2) gebildet wird,
- wobei der Messwert (F) eine Resonanzfrequenz des Parallelschwingkreises ist.

12. Verfahren nach einem der Ansprüche 7 bis 11,
- wobei in Abhängigkeit von dem Betriebszustand der Schleifkontakte (SK1, SK2) zumindest eines der zwei Schleifkontaktelemente (SE1, SE2) ausgetauscht wird.

13. Verfahren nach einem der Ansprüche 7 bis 12,
- wobei in Abhängigkeit von dem Betriebszustand der Schleifkontakte (SK1, SK2) ein Anpressdruck zumindest eines der zwei Schleifkontaktelemente (SE1, SE2) an die Schleifbahn (SB) verändert wird.

14. Verfahren nach einem der Ansprüche 7 bis 13,
- wobei der Messwert (F) erfasst wird, während mittels der zwei Schleifkontaktelemente (SE1, SE2) elektrische Energie zur Versorgung einer Röntgenquelle (26) von der Zuleitung (D) auf die Schleifbahn (SB) übertragen wird.

15. Verfahren nach Anspruch 14,
- wobei die elektrische Energie zur Versorgung der Röntgenquelle (26) in Form zumindest eines hochfrequenten Pulsstroms übertragen wird.

## Claims

1. Computed tomography device (1), including
- a slipring (SR) for rotational transmission,
- a feed line (D), wherein the slipring (SR) is arranged to be rotatable relative to the feed line (D),
- two sliding contact elements (SE1, SE2), which form sliding contacts (SK1, SK2) between a slideway (SB) of the slipring (SR) and the feed line (D) and which are connected in parallel with one another such that the two sliding contact elements (SE1, SE2) are electrically conductively connected together by means of at least one portion (L2) of the slideway (SB) and by means of at least one portion (L1) of the feed line (D),
- a measuring apparatus (MV), which is configured to acquire a measured value (F) which is dependent on the resultant inductance between the two sliding contact elements (SE1, SE2),
- a determination unit (EE), which is configured to determine an operating state of the sliding contacts (SK1, SK2) on the basis of the measured value (F).

2. Computed tomography device (1) according to claim 1,
- wherein the determination unit (EE) is configured to compare the measured value (F) with a reference value (R) and to determine the operating state of the sliding contacts (SK1, SK2) on the basis of a result of the comparison.

3. Computed tomography device (1) according to claim 1 or 2,
- wherein the measuring apparatus (MV) includes a capacitor which forms a parallel resonant circuit with the resultant inductance between the two sliding contact elements (SE1, SE2),
- wherein the measured value (F) is a resonant frequency of the parallel resonant circuit.

4. Computed tomography device (1) according to claim 3,
- wherein the measuring apparatus (MV) includes a coaxial cable (CX) and a resonant frequency determination unit which is configured to determine the resonant frequency of the parallel resonant circuit,
- wherein the two sliding contact elements (SE1, SE2) are connected by means of the coaxial cable (CX) to the resonant frequency determination unit in such a way that the coaxial cable (CX) forms at least one part of the capacitor, which, together with the resultant inductance between the two sliding contact elements (SE1, SE2), forms a parallel resonant circuit

5. Computed tomography device (1) according to claim 4,
- wherein the resonant frequency-determination unit is a network analyser (NA).

6. Computed tomography device (1) according to one of claims 1 to 5,
- wherein each of the two sliding contact elements (SE1, SE2) in each case takes the form of a carbon brush.

7. Method for determining an operating state of sliding contacts (SK1, SK2) on a slipring (SR) for rotational transmission in a computed tomography device (1), wherein the method comprises the following steps:
- acquiring (VF) a measured value (F) which is dependent on the resultant inductance between two sliding contact elements (SE1, SE2), which form the sliding contacts (SK1, SK2) between a slideway (SB) of the slipring (SR) and a feed line (D) and which are connected in parallel with one another in such a way that the two sliding contact elements (SE1, SE2) are electrically conductively connected together by means of at least one portion (L2) of the slideway (SB) and by means of at least one portion (L1) of the feed line (D), wherein the slipring (SR) is arranged so as to be rotatable relative to the feed line (D),
- determining (VS) the operating state of the sliding contacts (SK1, SK2) on the basis of the measured value (F).

8. Method according to claim 7,
- wherein the measured value (F) is compared with a reference value (R),
- wherein the operating state of the sliding contacts (SK1, SK2) is determined on the basis of a result of the comparison.

9. Method according to claim 7 or 8,
- wherein a time series of the measured value (F) and/or of a parameter determined on the basis of the measured value (F) is provided,
- wherein pattern recognition is performed on the basis of the time series,
- wherein the operating state of the sliding contacts (SK1, SK2) is determined on the basis of the result of the pattern recognition.

10. Method according to claim 9,
- wherein the pattern recognition is carried out on the basis of a trained machine learning algorithm.

11. Method according to one of claims 7 to 10,
- wherein a parallel resonant circuit is formed from a capacitor and the resultant inductance between the two sliding contact elements (SE1, SE2),
- wherein the measured value (F) is a resonant frequency of the parallel resonant circuit.

12. Method according to one of claims 7 to 11,
- wherein, as a function of the operating state of the sliding contacts (SK1, SK2), at least one of the two sliding contact elements (SE1, SE2) is replaced.

13. Method according to one of claims 7 to 12,
- wherein, as a function of the operating state of the sliding contacts (SK1, SK2), a contact pressure of at least one of the two sliding contact elements (SE1, SE2) against the slideway (SB) is modified.

14. Method according to one of claims 7 to 13,
- wherein the measured value (F) is acquired while electrical energy for supplying an X-ray source (26) is transmitted from the feed line (D) to the slideway (SB) by means of the two sliding contact elements (SE1, SE2).

15. Method according to claim 14,
- wherein the electrical energy for supplying the X-ray source (26) is transmitted in the form of at least one high frequency pulsed current.

## Revendications

1. Appareil (1) de tomodensitométrie à ordinateur, comportant
- un anneau (SR) de glissement de transmission d'une rotation,
- une ligne (D) d'amenée, l'anneau (SR) de glissement étant monté tournant par rapport à la ligne (D) d'amenée,
- deux éléments (SE1, SE2) de contact glissant, qui forment des contacts (SK1, SK2) glissants entre une voie (SB) de glissement de l'anneau (SR) de glissement et la ligne (D) d'amenée et qui sont montés parallèlement l'un à l'autre de manière à ce que les deux éléments (SE1, SE2) de contact glissant soient reliés l'un à l'autre d'une manière conductrice de l'électricité au moyen d'au moins un tronçon (L2) de la voie (SB) de glissement et au moyen d'au moins un tronçon (L1) de la ligne (D) d'amenée,
- un système (MV) de mesure, constitué pour relever une valeur (F) de mesure, qui dépend de l'induction résultante entre les deux éléments (SE1, SE2) de contact glissant,
- une unité (EE) de détermination, constituée pour déterminer un état de fonctionnement des contacts (SK1, SK2) glissants sur la base de la valeur (F) de mesure.

2. Appareil (1) de tomodensitométrie à ordinateur suivant la revendication 1,
- dans lequel l'unité (EE) de détermination est constituée pour comparer la valeur (F) de mesure à une valeur (R) de référence et pour déterminer l'état de fonctionnement des contacts (SK1, SK2) glissants sur la base d'un résultat de la comparaison.

3. Appareil (1) de tomodensitométrie à ordinateur suivant la revendication 1 ou 2,
- dans lequel le système (MV) de mesure a une capacité, qui forme un circuit oscillant parallèle avec l'inductance résultante entre les deux éléments (SE1, SE2) de contact glissant,
- dans lequel la valeur (F) de mesure est une fréquence de résonance du circuit oscillant parallèle.

4. Appareil (1) de tomodensitométrie à ordinateur suivant la revendication 3,
- dans lequel le système (MV) de mesure a un câble (CX) coaxial et une unité de détermination de la fréquence de résonance, qui est constituée pour déterminer la fréquence de résonance du circuit oscillant parallèle,
- dans lequel les deux éléments (SE1, SE2) de contact glissant sont reliés à l'unité de détermination de la fréquence de résonance au moyen du câble (CX) coaxial de manière à ce que le câble (CX) coaxial forme au moins une partie de la capacité, qui forme un circuit oscillant parallèle ensemble avec l'inductance résultante entre les deux éléments (SE1, SE2) de contact glissant.

5. Appareil (1) de tomodensitométrie à ordinateur suivant la revendication 4,
- dans lequel l'unité de détermination de la fréquence de résonnance est un analyseur (NA) de réseau.

6. Appareil (1) de tomodensitométrie à ordinateur suivant l'une des revendications 1 à 5,
- dans lequel chacun des deux éléments (SE1, SE2) de contact glissant est constitué, respectivement, sous la forme d'un balai de charbon.

7. Procédé de détermination d'un état de fonctionnement de contacts (SK1, SK2) glissants d'un anneau (SR) de glissement d'une transmission en rotation dans un appareil (1) de tomodensitométrie à ordinateur, le procédé comprenant les stades suivants :
- on relève (VF) une valeur (F) de mesure, qui dépend de l'inductance résultante entre deux éléments (SE1, SE2) de contact glissant, qui forme les contacts (SK1, SK2) glissants entre une voie (SB) de glissement de l'anneau (SR) de glissement et une ligne (D) d'amenée et qui sont montés en parallèle l'un à l'autre de manière à ce que les deux éléments (SE1, SE2) de contact glissant soient reliés l'un à l'autre d'une manière conductrice de l'électricité au moyen d'au moins un tronçon (L2) de la voie (SB) de glissement et au moyen d'au moins un tronçon (L1) de la ligne (D) d'amenée,
- on détermine (VS) l'état de fonctionnement des contacts (SK1, SK2) glissants sur la base de la valeur (F) de mesure.

8. Procédé suivant la revendication 7,
- dans lequel on compare la valeur (F) de mesure à une valeur (R) de référence,
- dans lequel on détermine l'état de fonctionnement des contacts (SK1, SK2) glissants sur la base d'un résultat de la comparaison.

9. Procédé suivant la revendication 7 ou 8,
- dans lequel on se procure une série dans le temps de la valeur (F) de mesure et/ou d'une valeur caractéristique que l'on détermine sur la base de la valeur (F) de mesure,
- dans lequel on effectue une reconnaissance de modèle reposant sur la série dans le temps,
- dans lequel on détermine l'état de fonctionnement des contacts (SK1, SK2) glissants sur la base d'un résultat de la reconnaissance de modèle.

10. Procédé suivant la revendication 9,
- dans lequel on effectue la reconnaissance de modèle en se basant sur un algorithme informatique à apprentissage.

11. Procédé suivant l'une des revendications 7 à 10,
- dans lequel on forme un circuit oscillant parallèle composé d'une capacité et de l'inductance résultante entre les deux éléments (SE1, SE2) de contact glissant,
- dans lequel la valeur (F) de mesure est la fréquence de résonance du circuit oscillant parallèle.

12. Procédé suivant l'une des revendications 7 à 11,
- dans lequel, en fonction de l'état de fonctionnement des contacts (SK1, SK2) glissants, on remplace au moins l'un des deux éléments (SE1, SE2) de contact glissant.

13. Procédé suivant l'une des revendications 7 à 12,
- dans lequel, en fonction de l'état de fonctionnement des contacts (SK1, SK2) glissants, on modifie la pression d'application d'au moins l'un des deux éléments (SE1, SE2) de contact glissant sur la voie (SB) de glissement.

14. Procédé suivant l'une des revendications 7 à 13,
- dans lequel on relève la valeur (F) de mesure pendant qu'au moyen des deux éléments (SE1, SE2) de contact glissant de l'énergie électrique d'alimentation d'une source (26) à rayons X est transportée de la ligne (D) d'amenée à la voie (SB) de glissement.

15. Procédé suivant la revendication 14,
- dans lequel on transporte l'énergie électrique d'alimentation de la source (26) à rayons X sous la forme d'au moins un courant pulsé à haute fréquence.
